(19) Europäisches Patentamt
European Patent Office
Office européen des brevets

(11) **EP 3 026 430 B1**

(12) **FASCICULE DE BREVET EUROPEEN**

(45) Date de publication et mention de la délivrance du brevet:
**10.10.2018 Bulletin 2018/41**

(51) Int Cl.:
*G01N 27/02* (2006.01)    *A61B 5/00* (2006.01)
*A61B 5/053* (2006.01)

(21) Numéro de dépôt: **15196407.9**

(22) Date de dépôt: **26.11.2015**

(54) **MÉTHODE D'IMAGERIE D'UN MILIEU PAR MESURES ÉLECTRIQUES AVEC CORRECTION D'IMPÉDANCE DE CONTACT**

BILDGEBENDES VERFAHREN FÜR EIN MEDIUM BASIEREND AUF ELEKTRISCHEN MESSUNGEN MIT KORREKTUR DER KONTAKTIMPEDANZ

METHOD FOR IMAGING A MEDIUM BY ELECTRICAL MEASUREMENTS WITH CONTACT IMPEDANCE CORRECTION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.11.2014 FR 1461677**

(43) Date de publication de la demande:
**01.06.2016 Bulletin 2016/22**

(73) Titulaires:
- **Commissariat à l'Énergie Atomique et aux Énergies Alternatives**
  **75015 Paris (FR)**
- **Institut National de la Santé et de la Recherche Médicale (INSERM)**
  **75013 Paris (FR)**

(72) Inventeurs:
- **FOUCHARD, Alexandre**
  **21000 Dijon (FR)**
- **BONNET, Stéphane**
  **69003 LYON (FR)**
- **DAVID, Olivier**
  **38700 LE SAPPEY (FR)**
- **PHAM, Pascale**
  **38920 CROLLES (FR)**

(74) Mandataire: **Brevalex**
**56, Boulevard de l'Embouchure**
**B.P. 27519**
**31075 Toulouse Cedex 2 (FR)**

(56) Documents cités:
**WO-A1-95/24155    WO-A1-98/23204**

- **HONG SUNJOO ET AL: "A 4.9 m[Omega]-Sensitivity Mobile Electrical Impedance Tomography IC for Early Breast-Cancer Detection System", IEEE JOURNAL OF SOLID-STATE CIRCUITS, IEEE SERVICE CENTER, PISCATAWAY, NJ, USA, vol. 50, no. 1, 29 septembre 2014 (2014-09-29), pages 245-257, XP011568755, ISSN: 0018-9200, DOI: 10.1109/JSSC.2014.2355835 [extrait le 2014-12-24]**
- **VILHUNEN T ET AL: "Detection of faults in resistive coatings with an impedance-tomography-related approach; Detection of faults in resistive coatings", MEASUREMENT SCIENCE AND TECHNOLOGY, IOP, BRISTOL, GB, vol. 13, no. 6, 7 mai 2002 (2002-05-07), pages 865-872, XP020063526, ISSN: 0957-0233, DOI: 10.1088/0957-0233/13/6/306**
- **EUN JUNG LEE ET AL: "Design of a microscopic electrical impedance tomography system for 3D continuous non-destructive monitoring of tissue culture", BIOMEDICAL ENGINEERING ONLINE, BIOMED CENTRAL LTD, LONDON, GB, vol. 13, no. 1, 6 octobre 2014 (2014-10-06), page 142, XP021199514, ISSN: 1475-925X, DOI: 10.1186/1475-925X-13-142**

- **WOO E J ET AL: "SKIN IMPEDANCE MEASUREMENTS USING SIMPLE AND COMPOUND ELECTRODES", MEDICAL AND BIOLOGICAL ENGINEERING AND COMPUTING, SPRINGER, HEILDELBERG, DE, vol. 30, no. 1, 2 janvier 1992 (1992-01-02), pages 97-102, XP000246244, ISSN: 0140-0118, DOI: 10.1007/BF02446200**

## Description

## DOMAINE TECHNIQUE

La présente invention concerne de manière générale le domaine de l'imagerie par mesures électriques et plus particulièrement celui de la tomographie d'impédance électrique.

La tomographie d'impédance électrique (ou TIE) est une technique d'imagerie non invasive, qui vise à reconstruire la distribution interne des propriétés électriques d'une zone d'un milieu à imager à partir de mesures électriques effectuées à sa surface ou sur son contour.

La tomographie d'impédance électrique a été originellement utilisée dans le domaine de la géophysique (où elle est plus connue sous le terme de « tomographie de résistivité électrique ») et plus particulièrement de l'exploration pétrolière, minière ou hydrologique. Elle a été ensuite appliquée au contrôle non destructif où elle est dénommée « tomographie de résistance électrique » ou « tomographie de capacitance électrique » selon qu'elle vise à reconstruire la conductivité électrique ou la permittivité électrique du milieu. Enfin, la tomographie d'impédance électrique a été appliquée avec succès au domaine médical à compter des années 1990. Elle a été notamment utilisée pour le suivi de la ventilation pulmonaire et du fonctionnement cérébral (détection et suivi des crises d'épilepsie), d'étude du fonctionnement du tractus gastro-intestinal, et le dépistage du cancer du sein. Malgré sa faible résolution spatiale, la TIE s'est développée ces dernières années en raison de son faible coût, son caractère non intrusif et non ionisant, sa grande résolution temporelle ainsi que son apport à la caractérisation des tissus et des fluides biologiques.

La tomographie d'impédance électrique suppose que l'on dispose à la surface de la zone d'intérêt du milieu à imager une pluralité d'électrodes. Ces électrodes permettent, d'une part, d'appliquer un signal électrique et donc un champ électrique dans le milieu en question et, d'autre part, de recueillir la réponse du milieu au signal ainsi appliqué.

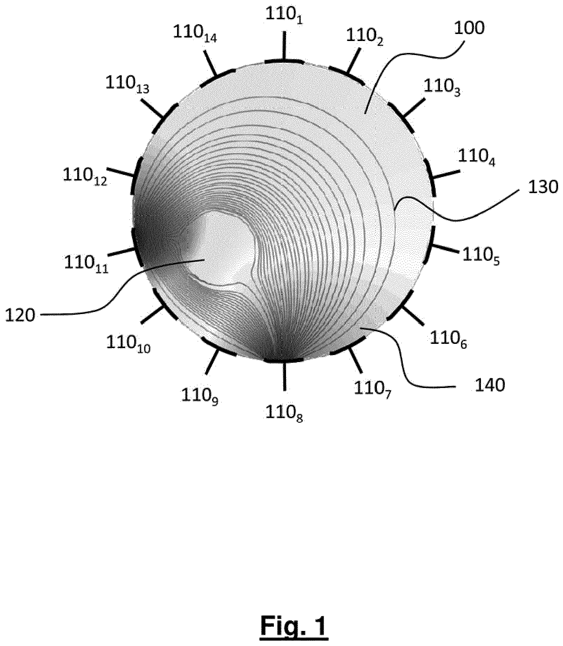

La Fig. 1 représente de manière schématique un dispositif expérimental de tomographie d'impédance électrique.

Le dispositif expérimental est ici, à titre d'illustration, un cylindre, représenté ici en coupe, délimitant une zone d'intérêt.

A la surface de la zone d'intérêt, 100, sont disposées N électrodes $110_1$, $110_2$, $110_3$,..., $110_N$ (ici $N$=14). On a supposé dans l'exemple illustré que la zone d'intérêt comprenait une anomalie cylindrique 120. Un courant est injecté dans le milieu (alternativement une différence de potentiel est appliquée à ce milieu) à l'aide de deux électrodes, on mesure alors la différence de potentiel (respectivement le courant) entre des paires d'électrodes quelconques. Dans l'exemple illustré, on injecte un courant dans le milieu au moyen des électrodes $100_8$ et $100_{11}$, et l'on mesure les différences de potentiel entre les électrodes $100_1$ et $100_2$, puis $100_2$ et $100_3$, et ainsi de suite. On a représenté sur la figure les lignes de courant par 130 et les équipotentielles par 140.

A partir des tensions (ou des courants) ainsi mesuré(e)s, on peut déduire et imager une cartographie d'une grandeur électrique, par exemple d'admittivité (conductivité complexe), la conductivité, la permittivité ou encore l'impédivité ou la résistivité dans la zone d'intérêt. On rappelle que l'admittivité (a*dmittivity*) $\tilde{\sigma}$ d'un milieu peut s'écrire sous la forme :

$$\tilde{\sigma} = \sigma + j\omega\varepsilon_0\varepsilon_r = 1/\tilde{z} \qquad (1)$$

où $\sigma$ est la conductivité du milieu, $\varepsilon = \varepsilon_r\varepsilon_0$ est la permittivité du milieu ($\varepsilon_0$ est la permittivité électrique du vide et $\varepsilon_r$ est la permittivité relative ou encore constante diélectrique du milieu) et $\tilde{z}$ est l'impédivité (*impedivity*) du milieu.

De manière générale, on pourra chercher à reconstruire l'équivalent d'une grandeur électrique tel qu'une quantité, ou une concentration, d'un constituant du milieu étudié, dont les propriétés électriques sont connues. Par exemple, une valeur de conductivité peut être aisément convertie en une quantité équivalente d'un matériau connu ayant cette conductivité. Par abus de langage, on désignera dans la suite par grandeur électrique les grandeurs électriques communément utilisées (impédivité, conductivité, permittivité, admittivité), ainsi que leurs équivalents.

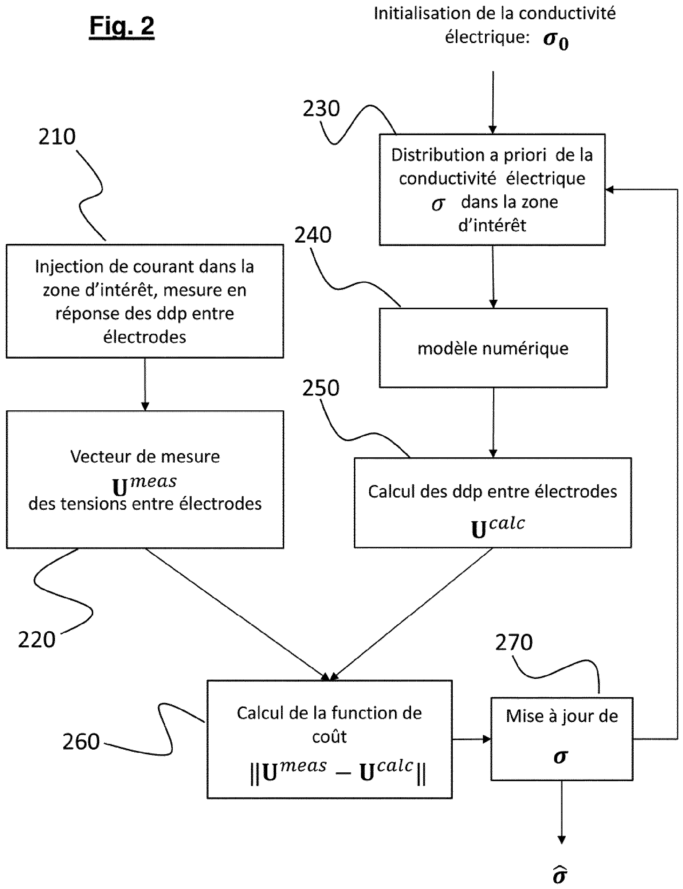

La Fig. 2 représente le schéma de principe d'une première méthode de tomographie d'impédance électrique connue de l'état de la technique.

Cette méthode, dite méthode de TIE absolue, fait appel à la résolution d'un premier problème dit « direct » et d'un second problème dit « inverse ».

Le problème direct utilise un maillage de la zone à imager. Il consiste à déterminer (de manière analytique ou par simulation numérique), à partir d'une hypothèse de distribution discrète de la grandeur électrique dans la zone d'intérêt, les signaux recueillis par les différentes électrodes (sous forme de différences de potentiel ou d'intensités de courant).

Le problème inverse consiste à déduire la cartographie de la grandeur électrique de la zone d'intérêt à partir des signaux effectivement observés, c'est-à-dire des signaux mesurés à l'aide des électrodes.

Plus précisément, la méthode de tomographie d'impédance représentée en Fig. 2 comprend une première étape 210 d'injection de courant dans la zone d'intérêt au moyen des électrodes et, en réponse, une mesure des différences de potentiel entre ces électrodes. Les différences de potentiel ainsi mesurées expérimentalement peuvent être rangées dans un vecteur de taille $N$, dit vecteur de mesure et noté $\mathbf{U}^{meas}$, fourni à l'étape 220.

Réciproquement, à partir d'une distribution spatiale donnée, $\sigma$, de la conductivité électrique dans la zone d'intérêt, 230, ($\sigma$ est un vecteur de taille $M$ où $M$ est le nombre de mailles de la zone d'intérêt), on peut calculer, en 240, à l'aide d'un modèle numérique, dit modèle direct, les différences de potentiel attendues lorsque les courants précités sont injectés. Ces différences de potentiel sont rangées en 250 dans un vecteur de taille $N$, dit vecteur calculé et noté $\mathbf{U}^{calc}$.

La méthode de TIE consiste à rechercher la distribution spatiale de conductivité électrique (ou de manière générale la distribution spatiale d'une grandeur électrique) dans la zone d'intérêt qui minimise une fonction de coût représentative de l'écart entre le vecteur mesuré et le vecteur calculé, $\|\mathbf{U}^{meas} - \mathbf{U}^{calc}\|$. La fonction de coût est calculée à l'étape 260.

Cette recherche ou résolution du problème inverse, est effectuée par itérations successives à partir d'une distribution initiale $\sigma_0$. Cette distribution initiale peut avoir été estimée par une autre méthode de caractérisation ou bien encore être une valeur de conductivité électrique moyenne dans la zone d'intérêt. Chaque itération permet de raffiner l'estimation (estimation *a priori*) de la conductivité électrique dans les différents points de la zone et l'estimation ainsi raffinée (estimation *a posteriori*) sert de nouvelle distribution *a priori* pour l'itération suivante. La mise à jour de la conductivité électrique est réalisée à l'étape 270.

De nombreux algorithmes peuvent être utilisés pour effectuer cette mise à jour, par exemple l'algorithme de Gauss-Newton. Un exemple de résolution du problème inverse est décrit dans l'article de L.M. Heikkinen et al. intitulé « Simultaneous reconstruction of electrode contact impedances and internai electrical properties : II. Laboratory experiments » publié dans Meas. Sci. Techno. 13 (2002), pp. 1855-1861.

Au terme du processus d'itération (nombre maximal d'itérations, détection de convergence), la méthode fournit une estimation de la distribution de conductivité électrique $\hat{\sigma}$ dans la zone considérée.

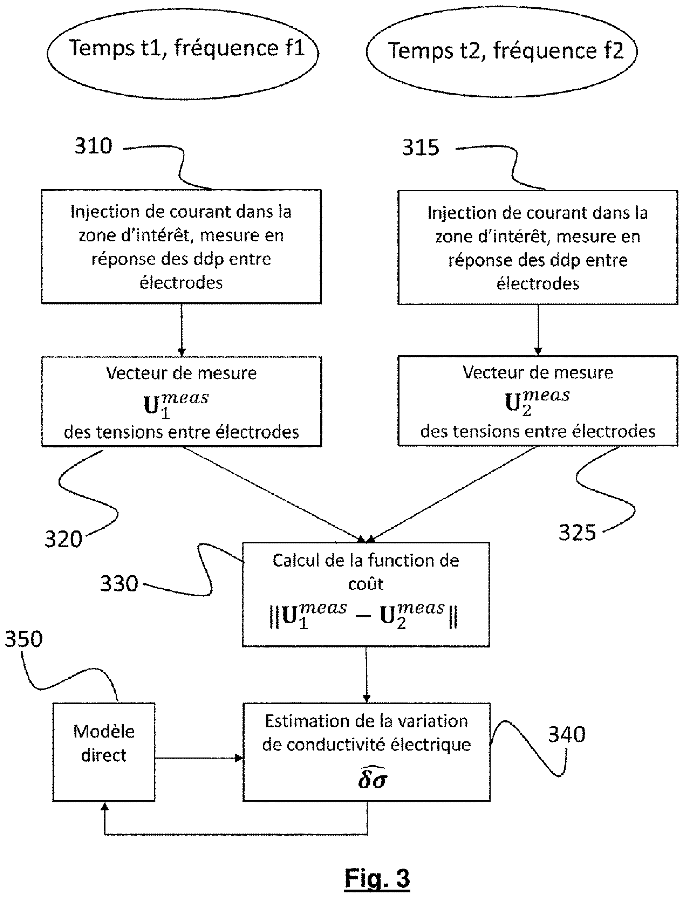

La Fig. 3 représente une seconde méthode de tomographie d'impédance connue de l'état de la technique. A la différence de la première celle-ci ne vise pas à obtenir une estimation de la conductivité électrique dans la zone d'intérêt mais simplement sa variation entre deux instants ou deux fréquences donné(e)s. Pour cette raison elle peut être qualifiée de méthode de TIE différentielle.

A partir du même dispositif expérimental, on injecte des courants d'intensité donnée dans une zone d'intérêt, aux étapes 310, 315 correspondant à deux instants différents ou à deux fréquences différentes. Les courants sont injectés au moyen des électrodes et l'on mesure au moyen de ces électrodes les différences de potentiel qui en résultent. On en déduit respectivement en 320, 325 les vecteurs de mesure $\mathbf{U}_1^{meas}$ et $\mathbf{U}_2^{meas}$ dont les éléments sont les différences de potentiel ainsi mesurées.

A l'étape 330, on calcule une fonction de coût représentative de l'écart $\left\| \mathbf{U}_1^{meas} - \mathbf{U}_2^{meas} \right\|$ entre les deux vecteurs de mesure et l'on recherche en 340 la distribution de variation de conductivité électrique, $\delta\sigma$, conduisant à cette variation de fonction de coût.

Pour ce faire, la fonction de coût est linéarisée autour du point de coordonnées ($_1$ , $\mathbf{U}_1^{meas}$) à partir du modèle direct, 350, où $\hat{\sigma}_1$ est la conductivité estimée à l'étape précédente.

La conductivité électrique $\hat{\sigma}_2$ permettant la linéarisation lors de la mesure suivante est obtenue par $\hat{\sigma}_2 = \hat{\sigma}_1 + \delta\sigma$. Toutefois, cette estimation est ici relativement grossière et ne sert qu'aux besoins de la linéarisation. Comme dans la méthode de TIE absolue, la référence initiale $\sigma_0$ peut être obtenue par une autre méthode de caractérisation ou bien encore être une valeur de conductivité électrique moyenne dans la zone d'intérêt.

La méthode de TIE différentielle ne permet d'estimer que l'évolution de la cartographie d'impédance entre deux instants différents ou entre deux fréquences différentes. Elle est moins sensible que la méthode de TIE absolue aux artefacts systématiques de mesure. De surcroît, elle ne requiert pas de connaître les positions des électrodes avec un degré de précision aussi élevé que pour la méthode de TIE absolue.

On trouvera une description exhaustive d'une méthode de TIE différentielle (en temps et en fréquence) dans l'ouvrage de J.K. Seo et E.J. Woo intitulé « Nonlinear inverse problems in imaging » Chap. 7.10-7.11, édité par J. Wiley & Sons.

Quel que soit le type de méthode de TIE, absolue ou différentielle, les différences de potentiel (respectivement les courants) en réponse à une injection de courant (respectivement une application de tensions) peuvent être mesurées selon différentes configurations de mesure.

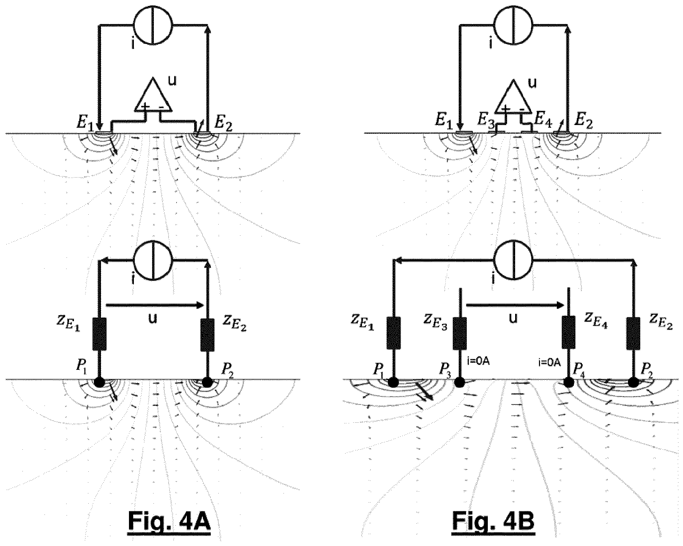

La Fig. 4A représente une première configuration de mesure pour la mise en oeuvre d'une méthode de TIE.

Cette première configuration, dite configuration deux points, est particulièrement simple puisque la mesure fait seulement appel à deux électrodes $E_1$ et $E_2$. Un courant $i$ est injecté dans le milieu au moyen de ces deux électrodes et l'on vient mesurer la différence de potentiel $u$ entre ces mêmes électrodes au moyen d'un amplificateur opérationnel.

Celui-ci présente en théorie une impédance infinie et donc un courant d'entrée nul qui ne perturbe pas la mesure. En revanche, comme représenté sur la partie basse de la figure, les impédances de contact des électrodes $E_1$ et $E_2$, respectivement désignées par $Z_{E_1}$ et $Z_{E_2}$, introduisent un biais dans la mesure en raison des chutes de potentiel intervenant entre leurs bornes. La différence de potentiel qui est mesurée en les électrodes $E_1$ et $E_2$ n'est donc pas représentative de la différence de potentiel entre les points $P_1$ et $P_2$ du milieu. Qui plus est, cette erreur de mesure est fonction du courant d'injection et de la fréquence. Elle est particulièrement importante pour des électrodes de petite taille (on parle alors de micro-TIE) puisque l'impédance de contact croît lorsque la surface de contact de l'électrode avec le milieu diminue. En pratique, les impédances de contact pour des électrodes de faible taille peuvent être supérieures à l'impédance du milieu situé entre deux électrodes.

La Fig. 4B représente une seconde configuration d'électrodes pour la mise en oeuvre d'une méthode de TIE.

Cette seconde configuration de mesure, dite configuration quatre points, fait appel à quatre électrodes, $E_1$ à $E_4$, deux électrodes $E_1, E_2$ servant à l'injection du courant dans le milieu et deux électrodes $E_3, E_4$, distinctes des premières, servant à la mesure de la différence de potentiel, en réponse à cette injection.

Dans cette configuration, l'impédance d'entrée de l'amplificateur opérationnel étant supposée infinie, aucun courant ne circule dans les impédances de contact $Z_{E_3}$ et $Z_{E_4}$ et, par conséquent, la différence de potentiel $u$ mesurée entre les électrodes $E_3$ et $E_4$ est bien celle présente entre les points $P_3$ et $P_4$ du milieu.

On comprend des figures 4A et 4B que seule la configuration de mesure à quatre points permet de s'affranchir des biais sur les différences de potentiel dues aux impédances de contact. Toutefois, pour un nombre d'électrodes donné, la configuration de mesure à quatre points peut conduire à un nombre de mesures indépendantes trop faibles et il est alors souhaitable de compléter le jeu de données en combinant une configuration à deux points et une configuration à quatre points. En effet, la résolution du problème inverse nécessite de disposer d'un grand nombre de mesures indépendantes en raison du nombre important d'inconnues ($M$ valeurs de la grandeur électrique discrétisée) à déterminer dans la résolution du problème inverse.

Pour obtenir un grand nombre de mesures sans que celles-ci soient entachées d'erreurs, la méthode classique propose d'intégrer dans le modèle direct les impédances de contact sous forme d'inconnues supplémentaires (en fait $N$ inconnues supplémentaires). La résolution du problème inverse implique alors la détermination conjointe des valeurs de la grandeur électrique et des impédances de contact.

Une première méthode de détermination conjointe des valeurs de la grandeur physique (aux différents points de la zone discrétisée) et des impédances de contact a été proposée dans l'article de L.M. Hekkinen précité ainsi que dans l'article associé de T. Vilhunen intitulé « Détection of faults in resistive coatings with an impedance-tomography-related approach » publié dans Meas. Sci. Technol. 13 (2002), pp. 865-872. Cette méthode suppose toutefois que le milieu de la zone d'intérêt soit homogène, hypothèse qui est rarement vérifiée en pratique, notamment pour les applications biomédicales.

Une seconde méthode de détermination conjointe des valeurs de la grandeur électrique et des impédances de contact a été proposée dans l'article de G. Boverman et al. intitulé « Methods for compensating for variable electrode contact in EIT » publié dans IEEE Trans. on Biomedical Engineering, vol. 56, No. 12, Dec. 2009, pp. 2762-2772. Cette méthode comprend une première étape dans laquelle deux paramètres sont estimés selon une approche non linéaire, à savoir une conductivité équivalente homogène et une impédance de contact moyenne valable pour toutes les électrodes, et une seconde étape d'estimation des valeurs de conductivité en chaque point ainsi que des différentes impédances de contact, par perturbation autour des paramètres précités.

Toutefois, cette méthode ne s'applique que dans le cas où l'on peut effectivement découpler le problème de la détermination des impédances de contact de celui des valeurs de la conductivité électrique. Or cette hypothèse n'est pas nécessairement vérifiée et, lorsqu'elle l'est, la méthode de détermination conjointe s'avère particulièrement complexe.

Une troisième méthode de détermination conjointe des valeurs de la grandeur physique et des impédances de contact a été proposée dans l'article de S. Hong intitulé « A 4.9 mΩ-Sensitivity Mobile Electrical Impedance Tomography IC for Early Breast-Cancer Détection System » publié dans le IEEE Journal of Solid-State Circuits (2015), pp. 245-257. Le but de la présente invention est, par conséquent, de proposer une méthode de TIE, et plus généralement d'imagerie d'une grandeur électrique dans une zone d'intérêt d'un milieu, permettant de s'affranchir, de manière simple et robuste, des erreurs dues aux impédances de contact des électrodes et ce, sans sacrifice de résolution dans cette zone d'intérêt.

## EXPOSÉ DE L'INVENTION

La présente invention est définie par une méthode de détermination de la distribution spatiale d'une grandeur électrique dans une zone d'intérêt d'un milieu, ladite méthode comprenant la réalisation de mesures de différences de potentiel entre électrodes dans ledit milieu, chaque électrode présentant avec ce milieu une surface de contact élémentaire, ladite méthode comprenant les étapes suivantes :

- on estime, pour chaque électrode, une impédance de contact avec ledit milieu ladite impédance de contact étant estimée en mesurant l'impédance entre ladite électrode et une contre-électrode présentant avec ledit milieu une surface de contact sensiblement supérieure à ladite surface de contact élémentaire ;
- on corrige les différences de potentiel à partir des chutes de potentiel intervenant dans les impédances de contact respectives desdites électrodes ;
- on détermine les valeurs de la grandeur électrique, ou d'une variation de celle-ci, en une pluralité de points de ladite zone d'intérêt, à partir des différences de potentiel ainsi corrigées.

L'invention concerne également une méthode de détermination de la distribution spatiale d'une grandeur électrique dans une zone d'intérêt d'un milieu, ladite méthode comprenant la réalisation de mesures de différences de potentiel au moyen d'électrodes disposées au contact de celui-ci, chaque électrode présentant avec ce milieu une surface de contact élémentaire, ladite méthode comprenant les étapes suivantes :

- on estime, pour chaque électrode, une impédance de contact avec ledit milieu, ladite impédance de contact étant estimée en mesurant l'impédance entre ladite électrode et une contre-électrode présentant avec ledit milieu une surface de contact sensiblement supérieure à ladite surface de contact élémentaire ;
- on construit un modèle direct corrigé permettant d'obtenir lesdites différences de potentiel à partir d'une distribution spatiale de la grandeur électrique dans la zone d'intérêt, ou d'une variation de celle-ci, le modèle direct corrigé prenant en compte les chutes de potentiel dans les impédances de contact ainsi estimées ;
- on détermine les valeurs de la grandeur électrique, ou de la variation de celle-ci, en une pluralité de points de ladite zone d'intérêt, à partir des mesures de différences de potentiel et du modèle direct corrigé.

Avantageusement, la contre-électrode est formée par un sous-ensemble de ladite pluralité d'électrodes, ledit sous-ensemble ne contenant pas l'électrode dont on mesure l'impédance de contact, les électrodes dudit sous-ensemble étant mises en court-circuit au moyen d'un commutateur à sortie unique.

Alternativement, la contre-électrode peut être une électrode dédiée, située à une distance des électrodes sensiblement supérieure à la distance moyenne entre ces dernières.

Avantageusement, l'impédance de contact peut être mesurée au moyen d'une spectroscopie d'impédance.

L'impédance de contact est avantageusement mesurée à partir d'un modèle de circuit équivalent représentant l'électrode et le milieu de la zone d'intérêt, l'impédance de contact étant celle dans le circuit équivalent permettant d'obtenir le spectre d'impédance le plus proche de celui mesuré par ladite spectroscopie d'impédance.

Ladite grandeur électrique peut être choisie parmi la conductivité, la résistivité, la permittivité, l'admittivité, l'impédivité du milieu, ou une fonction de l'une de ces grandeurs électriques, une quantité équivalente d'un matériau dont une grandeur électrique est connue, ou une fonction de cette quantité.

Selon une première variante, la variation de la grandeur électrique est prise en deux instants distincts.

Selon une seconde variante, la variation de la grandeur électrique est prise en deux fréquences distinctes.

Dans ce cas, ladite méthode peut être une méthode de TIE différentielle ou une méthode de TIE.

Avantageusement, les mesures de différences de potentiel entre électrodes sont effectuées en configuration deux points, un courant étant injecté entre deux électrodes et une différence de potentiel étant mesurée entre ces mêmes électrodes.

Quelle que soit le mode de réalisation, on peut ensuite construire une image de la grandeur électrique dans ce milieu, ou de sa variation, à partir des valeurs de la grandeur électrique précédemment déterminées.

## BRÈVE DESCRIPTION DES DESSINS

D'autres caractéristiques et avantages de l'invention apparaîtront à la lecture d'un mode de réalisation préférentiel de l'invention fait en référence aux figures jointes parmi lesquelles :

La Fig. 1 représente de manière schématique un dispositif expérimental de tomographie d'impédance électrique, connu de l'état de la technique ;
La Fig. 2 représente le schéma de principe d'une première méthode de tomographie d'impédance électrique connue de l'état de la technique ;
La Fig. 3 représente le schéma de principe d'une seconde méthode de tomographie d'impédance électrique connue de l'état de la technique ;
Les Figs. 4A et 4B représentent deux configurations de mesure pour une méthode de TIE ;
La Fig. 5 représente de manière schématique une méthode d'imagerie d'une grandeur électrique dans une zone d'intérêt, selon un premier mode de réalisation de l'invention ;
La Fig. 6 représente de manière schématique une méthode d'imagerie d'une grandeur électrique dans une zone d'intérêt, selon un second mode de réalisation de l'invention ;

La Fig. 7 représente de manière schématique une méthode d'imagerie d'une grandeur électrique dans une zone d'intérêt, selon un troisième mode de réalisation de l'invention ;

La Fig. 8 représente de manière schématique une méthode d'imagerie d'une grandeur électrique dans une zone d'intérêt, selon un quatrième mode de réalisation de l'invention ;

La Fig. 9 représente de manière schématique un système permettant la mesure des impédances de contact pour la méthode d'imagerie selon l'invention ;

La Fig. 10 représente schématiquement un circuit électrique équivalent à une impédance de contact ;

La Fig. 11 représente sous forme d'un diagramme de Bode un spectre d'impédance du circuit équivalent de la Fig. 9 en relation avec un spectre d'impédance entre une électrode et une contre-électrode ;

Les Figs. 12A et 12B représentent une image par TIE d'une zone d'intérêt d'un premier et d'un second milieux au moyen d'une méthode de TIE connue de l'état de la technique ;

Les Figs. 13A et 13B représentent une image par TIE de cette même zone d'intérêt au moyen d'une méthode de TIE selon un exemple de réalisation de l'invention.

## EXPOSÉ DÉTAILLÉ DE MODES DE RÉALISATION PARTICULIERS

On considérera par la suite une méthode d'imagerie d'une grandeur électrique dans la zone d'intérêt d'un milieu. La méthode d'imagerie fait appel à des mesures électriques de ce milieu au moyen d'une pluralité d'électrodes disposées à la surface de ce dernier. Dans la suite, nous supposerons, à titre d'illustration et sans préjudice de généralisation, que la méthode d'imagerie est une méthode de TIE.

L'idée à la base de l'invention est de déterminer l'impédance de contact de chaque électrode en utilisant une contre-électrode dont la surface est sensiblement plus importante que la surface de contact élémentaire entre ladite électrode et le milieu. La contre-électrode peut être avantageusement constituée par la mise en court-circuit d'un sous-ensemble de ladite pluralité d'électrodes ne contenant pas l'électrode dont l'impédance de contact est à mesurer.

Nous considérerons dans un premier temps une méthode de TIE absolue (ou TIE statique), dans la mesure où on estime la distribution d'une grandeur électrique au sein d'un milieu, par opposition à la TIE différentielle, où on estime la distribution de la variation d'une grandeur électrique au sein d'un milieu.

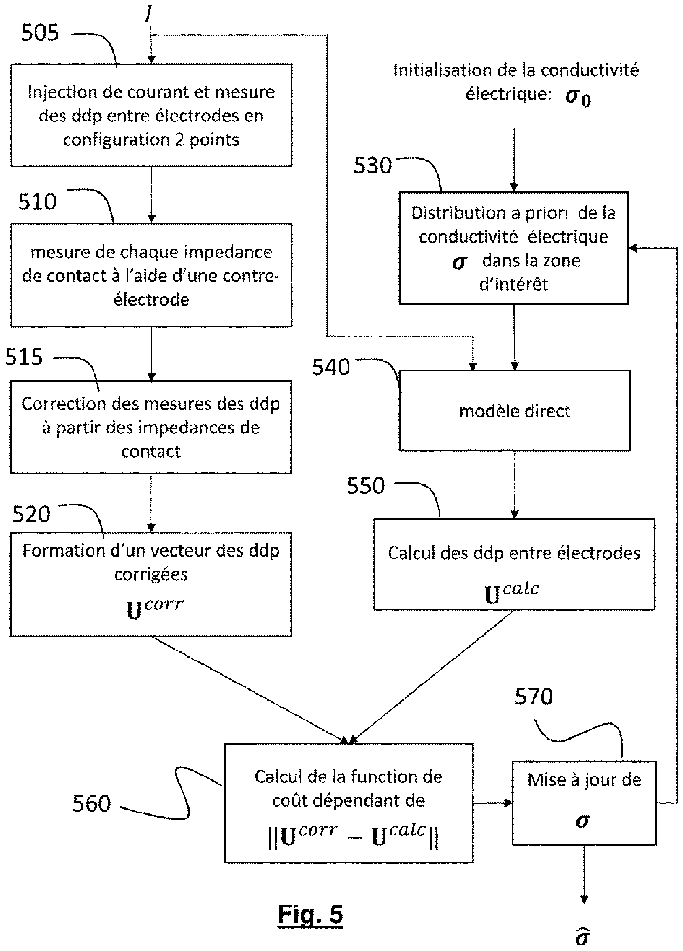

La Fig. 5 représente de manière schématique une méthode d'imagerie d'une grandeur électrique dans une zone d'intérêt, selon un premier mode de réalisation de l'invention.

A l'étape 505, on injecte un courant dans le milieu à l'aide d'une paire d'électrodes et l'on mesure la différence de potentiel entre ces mêmes électrodes selon une configuration de mesure à 2 points. Cette opération est répétée pour chaque paire d'électrodes de ladite pluralité d'électrodes. De manière plus générale, on pourra injecter un courant dans le milieu à l'aide d'un premier ensemble d'électrodes et mesurer les différences de potentiel entre des paires d'électrodes appartenant à un second ensemble d'électrodes, les premier et second ensembles d'électrodes n'étant pas disjoints.

A l'étape 510, on mesure l'impédance de contact de chaque électrode de ladite pluralité d'électrodes. Cette mesure est effectuée à l'aide d'une contre-électrode placée en configuration monopolaire, c'est-à-dire dont la présence n'influence pas la distribution du champ électrique au voisinage de l'électrode considérée. En théorie, cela correspond à une électrode placée à l'infini. En pratique, on réalise une approximation de la configuration monopolaire en adoptant, pour contre-électrode une électrode présentant une surface de contact avec le milieu sensiblement plus importante que la surface de contact élémentaire d'une électrode avec ce milieu. Par exemple, la surface de contact de la contre-électrode sera de l'ordre d'une dizaine voire de plusieurs dizaines ou plusieurs centaines de fois plus importante que la surface de contact élémentaire d'une simple électrode. Selon une première variante, la contre-électrode, CE, peut être une électrode dédiée, distincte des électrodes utilisées pour les mesures électriques du milieu. Dans ce cas-là, la contre-électrode sera avantageusement choisie distante de ces dernières. La distance moyenne entre les électrodes et la contre-électrode pourra être de l'ordre d'une dizaine voire une centaine de fois la distance moyenne inter-électrode.

Selon une seconde variante, la contre-électrode est réalisée en connectant ensemble un sous-ensemble d'électrodes de ladite pluralité d'électrodes. Plus précisément, si l'on désigne par $E_1,...,E_N$ ladite pluralité d'électrodes et $E_i$ l'électrode dont on souhaite déterminer l'impédance de contact, on met en court-circuit un sous-ensemble $\Omega_i$ d'électrodes $E_j, j \neq i,$ pour réaliser la contre-électrode $CE.$ De préférence, les électrodes du sous-ensemble $\Omega_i$ sont choisies distantes de l'électrode $E_i,$ au sens défini ci-dessus.

Le choix d'une contre-électrode distante, dédiée dans la première variante et composite dans la seconde variante, a pour but de ne pas influencer la mesure de l'impédance de contact par la distribution locale de la conductivité du milieu.

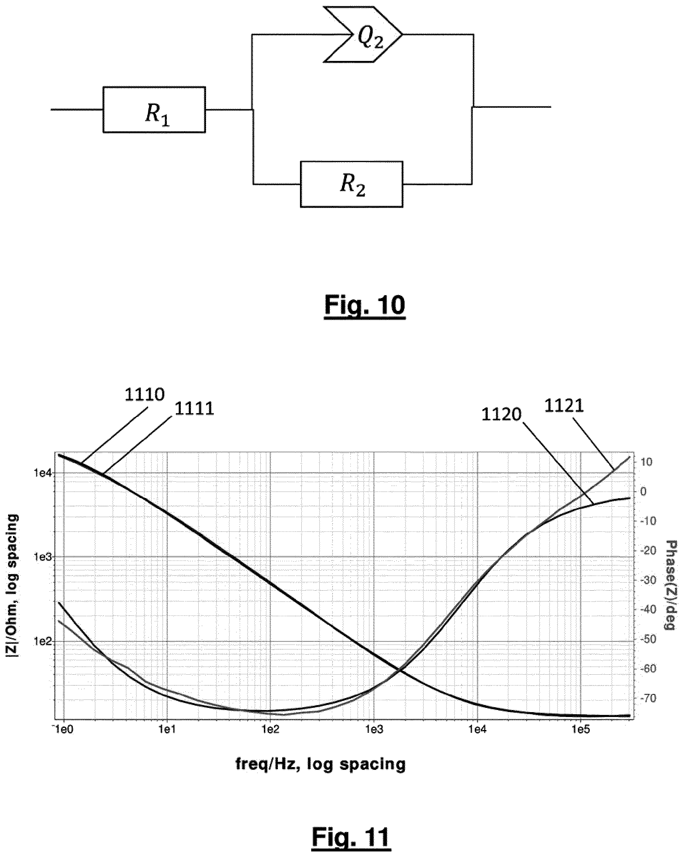

La mesure de l'impédance de contact d'une électrode est fondée sur une représentation de l'impédance entre l'électrode et la contre-électrode au moyen d'un circuit équivalent dont un exemple est décrit plus loin en relation avec la Fig. 10. Différents modèles de circuits équivalents plus ou moins complexes pourront être envisagés par l'homme du métier, selon le degré de précision souhaité, et selon la nature de l'électrode et des tissus biologiques avec lesquels elles sont en contact, sans pour autant sortir du cadre de la présente invention. Dans tous les cas, l'impédance de contact est déterminée à partir d'une spectroscopie d'impédance. Plus précisément, l'impédance complexe entre l'électrode et la contre-électrode est mesurée à une pluralité de fréquences. Il est alors possible de déterminer les impédances respectives des différents éléments du circuit équivalent, et notamment l'impédance de contact, de manière à ce que le spectre d'impédance du circuit équivalent soit le plus proche du spectre d'impédance mesuré au sens d'une certaine métrique.

Le spectre d'impédance peut être mesuré, soit en mode galvanostatique, en faisant circuler un courant prédéterminé entre l'électrode et la contre-électrode, et en mesurant la tension entre celles-ci, soit en mode potentiostatique, en appliquant une tension prédéterminée entre l'électrode et la contre-électrode, et mesurant le courant circulant de l'une à l'autre.

Quel que soit le mode de mesure utilisé, l'étape 510 fournit les impédances de contact des différentes électrodes. Il convient de noter que l'ordre des étapes 505 et 510 est indifférent.

A l'étape 515, on corrige les différences de potentiel mesurées à l'étape 505 à partir des impédances de contact mesurées à l'étape 510 et de la valeur du courant injecté. Plus précisément, si $u_{ij}$ est la différence de potentiel entre les électrodes $E_i$ et $E_j$, et si $Z_e^i$ et $Z_e^j$ sont les impédances de contact respectives des électrodes $E_i$ et $E_j$, la valeur corrigée $u_{ij}^{corr}$ de la différence de potentiel entre ces électrodes est :

$$u_{ij}^{corr} = u_{ij} - \left( Z_i^e + Z_j^e \right) I \qquad (2)$$

où $I$ est l'intensité du courant injecté. Le cas échéant l'intensité du courant injecté peut être choisie différente en fonction des paires d'électrodes considérées, auquel cas :

$$u_{ij}^{corr} = u_{ij} - \left( Z_i^e + Z_j^e \right) I_{ij} \qquad (3)$$

A l'étape 520, on forme le vecteur $\mathbf{U}^{corr}$ des différences de potentiel ainsi corrigées. Le vecteur $\mathbf{U}^{corr}$ est de taille $\dfrac{N(N-1)}{2}$, avec $\dfrac{N(N-1)}{2} \geq M$.

Par ailleurs, de la même manière qu'en Fig. 2, on effectue à partir d'une estimation *a priori* de la distribution de la conductivité électrique dans la zone d'intérêt, 530, et d'un modèle numérique (modèle direct) de la zone d'intérêt, 540, un calcul des différences de potentiels attendues entre les différentes électrodes, 550. Ce calcul est effectué en injectant dans le modèle direct l'intensité du courant injecté (ou selon le cas, les intensités des courants injectés) lors de la mesure à l'étape 505. Les différences de potentiel ainsi calculées sont rangées dans un vecteur $\mathbf{U}^{calc}$.

On calcule ensuite en 560 une fonction de coût dépendant de l'écart entre le vecteur des différences de potentiel calculées et le vecteur des différences de potentiel corrigées. Cette fonction de coût peut aussi comprendre un terme de régularisation fonction de l'écart entre la distribution estimée de la conductivité, $\hat{\sigma}$ et d'une distribution de référence, $\sigma^{ref}$.

L'estimation de la distribution de la conductivité est mise à jour de manière itérative, en 570, à partir d'une distribution initiale, $\sigma_0$, de manière à minimiser la fonction de coût, par exemple :

$$f_h(\widehat{\boldsymbol{\sigma}}) = \frac{1}{2}\left( \left\| \mathbf{U}^{corr} - \mathbf{U}^{calc} \right\|^2 + h^2 \|\widehat{\boldsymbol{\sigma}}\|^2 \right) \qquad (4)$$

Cette mise à jour peut être effectuée au moyen d'un algorithme classique de Gauss-Newton ou de Newton-Raphson, de manière connue en soi.

Ainsi, pour chaque itération $l$, la conductivité à l'étape $l + 1$ est déterminée par :

$$\boldsymbol{\sigma}^{l+1} = \boldsymbol{\sigma}^l + \delta\boldsymbol{\sigma}^l \qquad (5\text{-}1)$$

où l'incrément $\delta\sigma^l$ est déterminé par :

$$\delta\boldsymbol{\sigma}^{l} = (\mathbf{J}^{T}\mathbf{J} + h^{2}\mathbf{R})^{-1}\mathbf{J}^{T}\delta\mathbf{U} \qquad\qquad (5\text{-}2)$$

où $\delta\mathbf{U} = \mathbf{U}^{corr} - \mathbf{U}^{calc}$, $\mathbf{R}$ est une matrice de régularisation, par exemple la matrice identité, $\mathbf{J}$ est la matrice jacobienne de $\mathbf{U}$ par rapport à $\sigma$ et $h$ un hyperparamètre qui contrôle le compromis entre le terme d'attache aux données et le terme de régularisation dans la fonction de coût.

L'homme du métier pourra compléter ou adapter cette approche, avec par exemple la prise en compte de matrices de pondération sur les données et sur l'espace, et envisager d'autres approches d'estimation de paramètres ne mettant pas en oeuvre la matrice jacobienne $\mathbf{J}$, par exemple des méthodes analytiques ou des méthodes de type D-bar.

La distribution de la conductivité ainsi estimée dans la zone d'intérêt peut ensuite être représentée sous forme d'image.

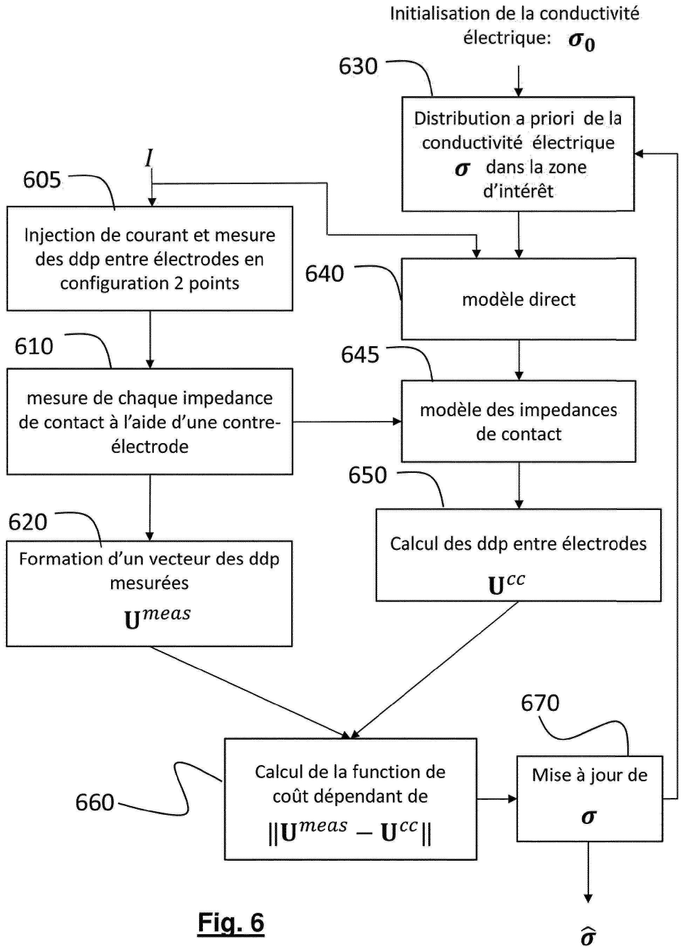

La Fig. 6 représente de manière schématique une méthode d'imagerie d'une grandeur électrique dans une zone d'intérêt, selon un second mode de réalisation de l'invention.

A la différence du mode de réalisation précédent, les différences de potentiel ne sont pas corrigées des chutes de potentiel dans les électrodes lors de la mesure mais cette correction est prise en compte dans le modèle direct.

Les étapes 605 et 610 sont respectivement identiques aux étapes 505 et 510 précédemment décrites. En revanche, les impédances de contact mesurées en 610 ne servent pas ici à corriger les différences de potentiel mesurées mais sont transmises à l'étape 645 pour corriger le modèle direct. Les mesures des différences de potentiel sont rangées sans correction dans un vecteur noté $\mathbf{U}^{meas}$.

De manière similaire, les étapes 630 et 640 sont respectivement identiques aux étapes 530 et 540. Les différences de potentiel issues du modèle direct sont corrigées en 645 au moyen des impédances de contact mesurées à l'étape 610. Plus précisément, si les différences de potentiel calculées à l'aide du modèle direct sont notées $u_{ij}^{calc}$, les différences de potentiel corrigées en prenant en compte les impédances de contact sont obtenues par :

$$u_{ij}^{cc} = u_{ij}^{calc} - \left(Z_{i}^{e} + Z_{j}^{e}\right)I \qquad\qquad (6)$$

Les différences de potentiel ainsi corrigées sont rangées dans un vecteur $\mathbf{U}^{cc}$ en 660.

La distribution spatiale de la conductivité dans la zone d'intérêt est ensuite estimée au moyen d'un processus itératif visant à minimiser une fonction de coût, 660, dépendant de l'écart $\lVert\mathbf{U}^{meas} - \mathbf{U}^{cc}\rVert$ entre les différences de potentiels mesurées et celles calculées en tenant compte de la correction due aux impédances de contact. Comme dans le premier mode de réalisation, la distribution spatiale de la conductivité ainsi estimée peut être représentée sous forme d'image.

Selon une variante non représentée, le modèle direct intègre les impédances de contact, autrement dit les valeurs de ces impédances sont des paramètres du modèle au même titre que les valeurs de la conductivité électrique dans les différentes mailles de la zone d'intérêt. Dans ce cas, on comprendra que les étapes 640 et 645 sont alors fusionnées.

La distribution de la conductivité ainsi estimée en 670 dans la zone d'intérêt peut ensuite être représentée sous forme d'image.

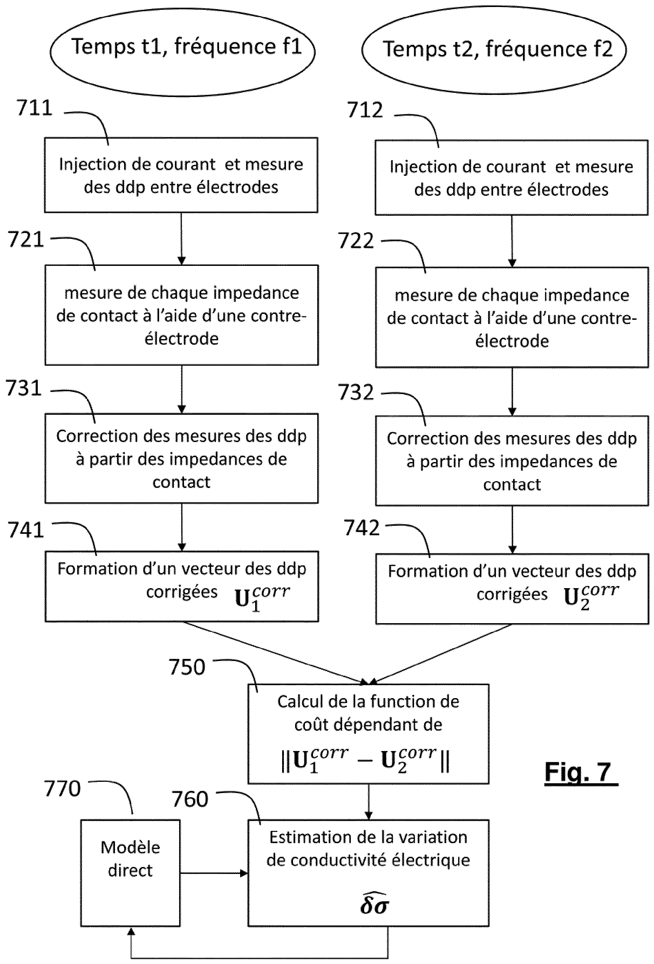

La Fig. 7 représente de manière schématique une méthode d'imagerie d'une grandeur électrique dans une zone d'intérêt, selon un troisième mode de réalisation de l'invention.

Ce mode de réalisation est relatif par exemple à une méthode de TIE différentielle.

A un temps $t_1$ (ou à une fréquence $f_1$) on effectue en 711 une injection de courant entre deux électrodes et l'on mesure la différence de potentiel entre ces mêmes électrodes. Cette opération est répétée pour chaque paire d'électrodes.

On mesure ensuite à l'étape 721, l'impédance de contact de chacune de ces électrodes (au temps $t_1$ ou à la fréquence $f_1$). Cette mesure est réalisée comme aux étapes 510 et 610, à l'aide d'une contre-électrode de grande surface par rapport à une surface de contact élémentaire d'une électrode ou bien en court-circuitant un sous-ensemble d'électrodes ne contenant pas l'électrode dont on souhaite mesurer l'impédance de contact.

A l'étape 731, on corrige les différences de potentiel mesurées à l'étape 711 au moyen des impédances de contact déterminées à l'étape 721. Les différences de potentiel ainsi corrigées sont rangées dans un premier vecteur $\mathbf{U}_{1}^{corr}$.

Les étapes 711-741 sont répétées à un second instant $t_2$ ou à une seconde fréquence $f_2$, respectivement aux étapes 712-742. En particulier, on comprend que les impédances de contact sont aussi mesurées en ce second instant ou cette seconde fréquence. Les mesures des différences de potentiel corrigées sont rangées dans un second vecteur $\mathbf{U}_{2}^{corr}$.

A l'étape 750, on détermine la valeur d'une fonction de coût dépendant de l'écart $\left\| \mathbf{U}_1^{corr} - \mathbf{U}_2^{corr} \right\|$ entre le premier vecteur des mesures corrigées et le second vecteur des mesures corrigées. On comprend ainsi que les variations de différences de potentiel sont débarrassées de la contribution due à la variation des impédances de contact entre les deux instants ou les deux fréquences.

Comme à l'étape 340 de la méthode de TIE différentielle en Fig. 3, la fonction de coût est linéarisée autour du point de coordonnées $\left( \hat{\boldsymbol{\sigma}}_1 , \mathbf{U}_1^{corr} \right)$ à partir du modèle direct, 770, où $\hat{\sigma}_1$ est la conductivité estimée à l'étape précédente.

La variation de conductivité $\widehat{\boldsymbol{\delta\sigma}}$ est obtenue en 760 à partir de la fonction de coût linéarisée et la distribution conductivité de référence $\hat{\sigma}_1$ est mise à jour par $\hat{\sigma}_2 = \hat{\sigma}_1 + \delta\sigma$ pour l'application du modèle direct.

La variation de conductivité entre deux instants ou deux fréquences pour la TIE différentielle peut être déterminée par $\delta\sigma = (\mathbf{J}^T\mathbf{J} + h^2\mathbf{R})^{-1}\mathbf{J}^T\delta\mathbf{U}$ où $\boldsymbol{\delta\mathbf{U}} = \mathbf{U}_2^{corr} - \mathbf{U}_1^{corr}$ pour ce mode de réalisation de l'invention.

L'homme du métier pourra compléter ou adapter cette approche, avec par exemple la prise en compte de matrices de pondération sur les données et sur l'espace, et envisager d'autres approches d'estimation de paramètres qui ne mettent pas en oeuvre la matrice Jacobienne **J,** par exemple des méthodes analytiques ou des méthodes D-bar.

La distribution spatiale de la variation de conductivité peut ensuite être représentée sous forme d'une image de la zone d'intérêt.

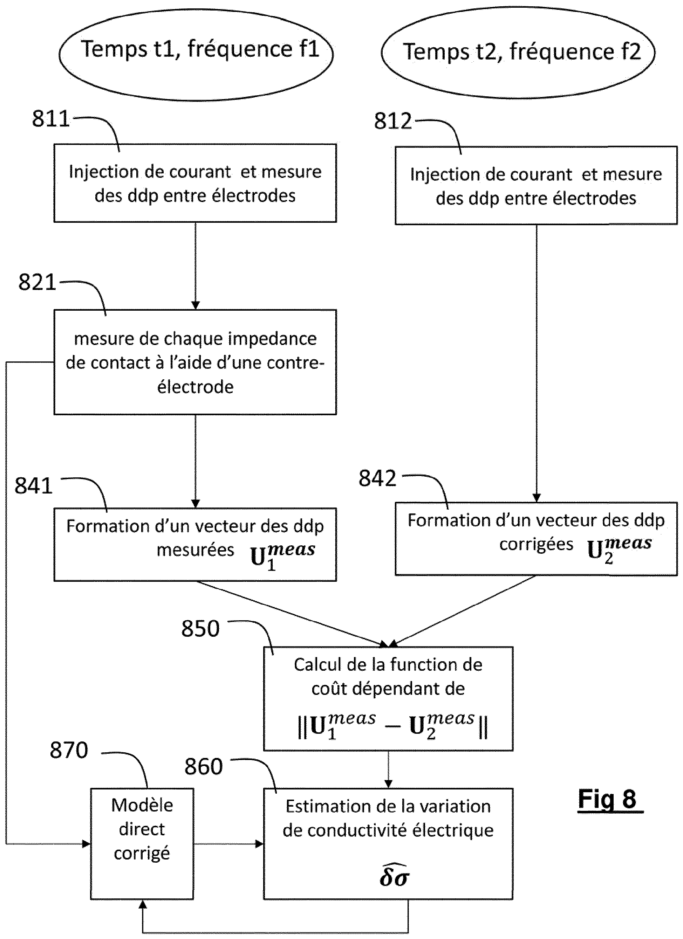

La Fig. 8 représente de manière schématique une méthode d'imagerie d'une grandeur électrique dans une zone d'intérêt, selon un quatrième mode de réalisation de l'invention.

Ce mode de réalisation est, comme le précédent, de type différentiel dans le sens où en deux instants consécutifs, ou pour deux fréquences différentes, on effectue une injection de courant et une mesure de différence de potentiel pour chaque paire l'électrodes, au moyen d'une configuration 2 points. Les étapes 811 et 812 sont identiques aux étapes 711 et 712 de la Fig. 7. Au premier instant, ou pour la première fréquence, on effectue une mesure des impédances de contact des électrodes en 821, de la même façon qu'à l'étape 721.

Toutefois dans le présent mode de réalisation, les mesures de différences de potentiel ne sont pas corrigées des chutes de potentiel dans les impédances de contact. Les différences de potentiel au premier instant/ première fréquence et au second instant/ seconde fréquence sont respectivement rangées en 841 et 842 dans les vecteurs $\mathbf{U}_1^{meas}$ et $\mathbf{U}_2^{meas}$.

A l'étape 850, on détermine la valeur d'une fonction de coût dépendant de l'écart $\left\| \mathbf{U}_1^{meas} - \mathbf{U}_2^{meas} \right\|$ entre le premier vecteur des mesures corrigées et le second vecteur des mesures corrigées. Cette fonction de coût est linéarisée autour du point $\left( \hat{\boldsymbol{\sigma}}_1 , \mathbf{U}_1^{meas} \right)$, à partir du modèle direct corrigé, 870, pour prendre en compte les impédances de contact déterminées à l'étape 821.

On détermine ainsi en 860 la distribution spatiale de la variation de conductivité électrique dans la zone d'intérêt, $\widehat{\boldsymbol{\delta\sigma}}$. Cette distribution spatiale peut être représentée sous forme d'une image de la zone d'intérêt.

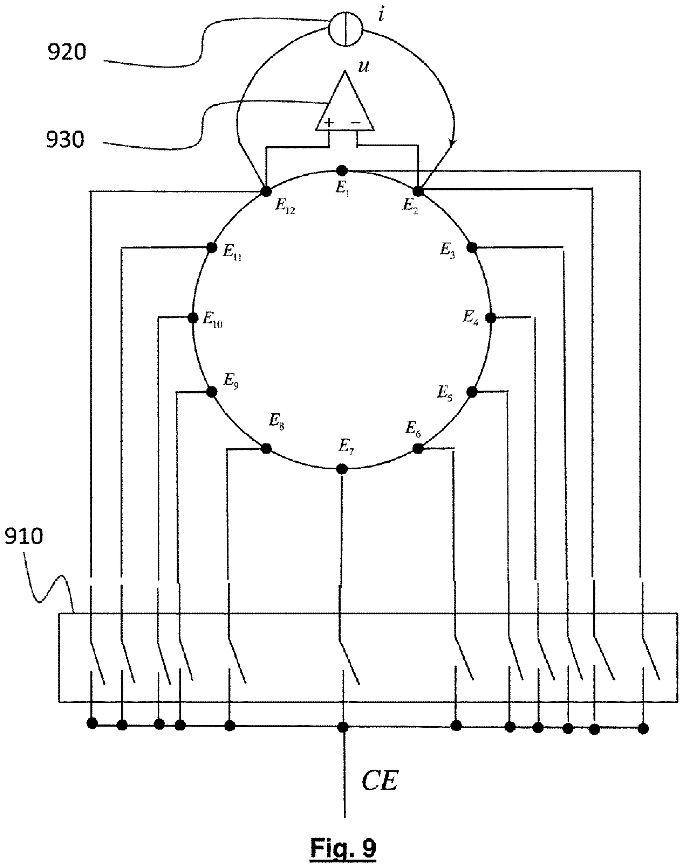

La Fig. 9 représente un système permettant la mesure des impédances de contact pour les modes de réalisation de l'invention illustrés dans les Figs. 5-8. Autrement dit ce système peut être utilisé dans les étapes 510, 610, 721-722 et 821. Les différentes électrodes $E_i$, $i = 1,...,N$, sont reliées à un commutateur, 910, à $N$ entrées et une sortie commune *CE*, chaque entrée pouvant être ou non commutée sur la sortie commune.

Lorsque l'on effectue un mesure de différence de potentiel *u* entre deux électrodes $E_i$ et $E_j$, on relie ces électrodes aux bornes d'un générateur de courant 920 injectant un courant *i* dans le milieu. La différence de potentiel *u* est mesurée à l'aide d'un amplificateur opérationnel 930. Dans ce mode de mesure, les entrées du commutateur 910 sont déconnectées de la sortie commune.

En revanche, lorsque l'on souhaite mesurer l'impédance de contact d'une électrode $E_i$, on choisit un sous-ensemble $\Omega_i$ d'électrodes de $\Omega$ ne contenant pas $E_i$. Les électrodes de $\Omega_i$ sont de préférence choisies éloignées de $E_i$, par exemple telles que leur distance à $E_i$ soit de l'ordre d'une dizaine à plusieurs dizaines de fois la distance moyenne entre électrodes voisines. Les électrodes de $\Omega_i$ sont connectées à la sortie commune *CE* alors que les électrodes de $\Omega\backslash\Omega_i$ n'y sont pas connectées. Le générateur de courant 920 est relié à l'électrode $E_i$ d'une part et à la sortie commune du commutateur *CE* d'autre part. De même, les bornes d'entrée de l'amplificateur opérationnel 930 sont aussi connectées à $E_i$ et *CE.* On mesure ainsi l'impédance d'un circuit équivalent constitué de l'impédance de contact de l'électrode $E_i$ en série avec l'impédance du milieu.

La Fig. 10 représente un exemple de circuit équivalent modélisant l'impédance entre une électrode et la contre-électrode *CE*.

Dans cet exemple, le circuit équivalent est obtenu par la mise en série d'une première résistance $R_1$, modélisant le comportement propre du milieu dans la partie haute fréquence du spectre (fréquences supérieures à 50 KHz) et de l'impédance de contact de l'électrode, décrivant les phénomènes d'interface dans la partie basse fréquence du spectre (fréquences inférieures à 10 kHz).

L'impédance de contact peut être modélisée par une seconde résistance $R_2$ en parallèle avec un élément à

$$Z_e(\omega) = \frac{R_2}{R_2 Q_2 (j\omega)^{\alpha_2} + 1}.$$

phase constante de paramètres $Q_2$ et $\alpha_2$. L'impédance de contact à la pulsation $\omega$ est alors

En effectuant une mesure du spectre d'impédance entre l'électrode d'intérêt et la contre-électrode, on peut trouver les valeurs des éléments électriques du circuit équivalent qui permettent de se rapprocher au mieux de l'impédance de contact de l'électrode d'intérêt.

Par exemple, la Fig. 11 représente selon un diagramme de Bode, le spectre d'impédance mesuré entre une électrode d'intérêt et la contre-électrode (module en 1111 et phase en 1121) ainsi que le spectre d'impédance du circuit équivalent de la Fig. 10, approchant au plus près celui mesuré (module de l'impédance en 1110 et phase de l'impédance en 1120). En particulier, on peut ainsi déterminer les éléments $R_2$ et $Q_2$ modélisant l'impédance de contact.

A partir des paramètres du modèle (ici $R_2$ et $Q_2$), on détermine l'impédance de contact complexe, ou son module, ou sa phase, ou sa partie réelle, ou sa partie imaginaire.

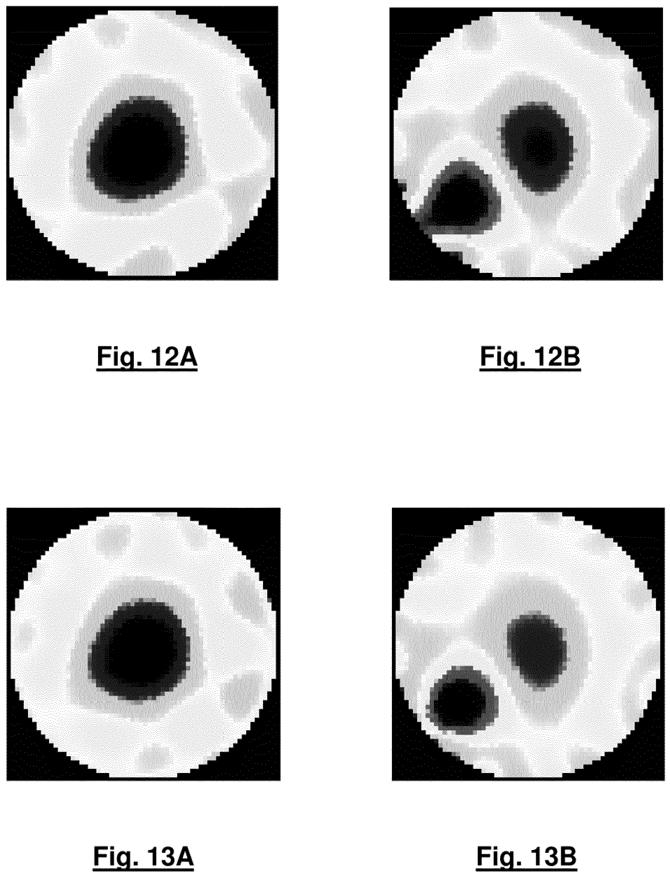

Les Figs. 12A et 12B représentent une image de TIE différentielle en fréquence correspondant à deux échantillons différents.

L'échantillon de la Fig. 12A présente une distribution de conductivité ayant une symétrie de révolution autour de l'axe central de l'échantillon.

L'échantillon de la Fig. 12B présente une distribution de conductivité décentrée par rapport à l'axe central de l'échantillon.

Pour ces deux échantillons, la méthode d'imagerie TIE différentielle utilisée fait appel à des mesures de différences de potentiel à l'aide d'une configuration de mesure à 4 points. Les impédances de contact ont été considérées égales à une valeur empirique.

Les Figs. 13A et 13B représentent une image de TIE différentielle en fréquence selon le troisième mode de réalisation de l'invention, pour les mêmes échantillons que ceux représentés en Figs. 12A et 12B.

Les différences de potentiel ont été mesurées en configuration 2 points, les mêmes électrodes servant à l'injection de courant et à la mesure de tension.

Les impédances de contact ont été mesurées par spectroscopie d'impédance à partir de la modélisation de la Fig. 10.

On remarque que les images des Figs. 13A et 13B sont mieux résolues que celles des Figs. 12A et 12B et présentent moins d'artefacts. En particulier la zone de conductivité décentrée en Fig. 13B est mieux isolée du bord de la zone d'intérêt.

L'invention présente ainsi une alternative à l'état de l'art pour la prise en compte des impédances de contact dans le processus de TIE. L'ensemble des électrodes peut ainsi être utilisé pour l'acquisition des données, puisque le lien entre les mesures et les données est correctement effectué. Cela augmente alors le nombre de mesures indépendantes disponibles à nombre d'électrodes fixé. En particulier, cela permet de mettre en oeuvre des configurations de mesure dans lesquelles au moins une des électrodes injectant du courant dans le milieu étudié est utilisée pour réaliser une mesure de différence de potentiel. On améliore ainsi alors la fiabilité de la reconstruction.

**Revendications**

1. Méthode de détermination de la distribution spatiale d'une grandeur électrique dans une zone d'intérêt d'un milieu, ladite méthode comprenant la réalisation de mesures de différences de potentiel entre électrodes dans ledit milieu, chaque électrode présentant avec ce milieu une surface de contact élémentaire, **caractérisée en ce que** :

    - on estime, pour chaque électrode, une impédance de contact avec ledit milieu (510,721,722) ladite impédance de contact étant estimée en mesurant l'impédance entre ladite électrode et une contre-électrode présentant avec ledit milieu une surface de contact sensiblement supérieure à ladite surface de contact élémentaire ;
    - on corrige (515,731,732) les différences de potentiel à partir des chutes de potentiel intervenant dans les impédances de contact respectives desdites électrodes ;
    - on détermine (560,760) les valeurs de la grandeur électrique, ou d'une variation de celle-ci, en une pluralité de points de ladite zone d'intérêt, à partir des différences de potentiel ainsi corrigées.

**2.** Méthode de détermination de la distribution spatiale d'une grandeur électrique dans une zone d'intérêt d'un milieu, ladite méthode comprenant la réalisation de mesures de différences de potentiel au moyen d'électrodes disposées au contact de celui-ci, chaque électrode présentant avec ce milieu une surface de contact élémentaire, **caractérisée en ce que** :

- on estime, pour chaque électrode, une impédance de contact avec ledit milieu (610, 821), ladite impédance de contact étant estimée en mesurant l'impédance entre ladite électrode et une contre-électrode présentant avec ledit milieu une surface de contact sensiblement supérieure à ladite surface de contact élémentaire ;
- on construit un modèle direct corrigé permettant d'obtenir lesdites différences de potentiel à partir d'une distribution spatiale de la grandeur électrique dans la zone d'intérêt, ou d'une variation de celle-ci, le modèle direct corrigé prenant en compte les chutes de potentiel dans les impédances de contact ainsi estimées ;
- on détermine (660, 860) les valeurs de la grandeur électrique, ou de la variation de celle-ci, en une pluralité de points de ladite zone d'intérêt, à partir des mesures de différences de potentiel et du modèle direct corrigé.

**3.** Méthode de détermination de la distribution spatiale d'une grandeur électrique selon la revendication 1 ou 2, **caractérisée en ce que** la contre-électrode est formée par un sous-ensemble de ladite pluralité d'électrodes, ledit sous-ensemble ne contenant pas l'électrode dont on mesure l'impédance de contact, les électrodes dudit sous-ensemble étant mises en court-circuit au moyen d'un commutateur (910) à sortie unique.

**4.** Méthode de détermination de la distribution spatiale d'une grandeur électrique selon la revendication 1 ou 2, **caractérisée en ce que** la contre-électrode est une électrode dédiée à une distance des électrodes sensiblement supérieure à la distance moyenne entre ces dernières.

**5.** Méthode de détermination de la distribution spatiale d'une grandeur électrique selon l'une des revendications précédentes, **caractérisée en ce que** l'impédance de contact est mesurée au moyen d'une spectroscopie d'impédance.

**6.** Méthode de détermination de la distribution spatiale d'une grandeur électrique selon la revendication 5, **caractérisée en ce que** l'impédance de contact est mesurée à partir d'un modèle de circuit équivalent représentant l'électrode et le milieu de la zone d'intérêt, l'impédance de contact étant celle dans le circuit équivalent permettant d'obtenir le spectre d'impédance le plus proche de celui mesuré par ladite spectroscopie d'impédance.

**7.** Méthode de détermination de la distribution spatiale d'une grandeur électrique selon l'une des revendications précédentes, **caractérisée en ce que** ladite grandeur électrique est choisie parmi la conductivité, la résistivité, la permittivité, l'admittivité, l'impédivité du milieu, ou une fonction de l'une de ces grandeurs électriques, une quantité équivalente d'un matériau dont une grandeur électrique est connue, ou une fonction de cette quantité.

**8.** Méthode de détermination de la distribution spatiale d'une grandeur électrique selon l'une des revendications précédentes, **caractérisée en ce que** la variation de la grandeur électrique est prise en deux instants distincts.

**9.** Méthode de détermination de la distribution spatiale d'une grandeur électrique selon l'une des revendications précédentes, **caractérisée en ce que** la variation de la grandeur électrique est prise en deux fréquences distinctes.

**10.** Méthode de détermination de la distribution spatiale d'une grandeur électrique selon l'une des revendications 8 ou 9, **caractérisée en ce qu'**elle est une méthode de TIE différentielle.

**11.** Méthode de détermination de la distribution spatiale d'une grandeur électrique selon l'une des revendications 1 à 7, **caractérisée en ce qu'**elle est une méthode de TIE.

**12.** Méthode de détermination de la distribution spatiale d'une grandeur électrique selon l'une des revendications précédentes, **caractérisée en ce que** les mesures de différences de potentiel entre électrodes sont effectuées en configuration deux points, un courant étant injecté entre deux électrodes et une différence de potentiel étant mesurée entre ces mêmes électrodes.

**13.** Méthode de détermination de la distribution spatiale d'une grandeur électrique selon l'une des revendications précédentes, **caractérisée en ce que** l'on construit une image de la grandeur électrique dans ce milieu, ou de sa variation, à partir des valeurs de la grandeur électrique précédemment déterminées.

**Patentansprüche**

1. Verfahren zum Bestimmen der räumlichen Verteilung einer elektrischen Größe in einem betreffenden Bereich eines Mediums, wobei das Verfahren das Durchführen von Messungen von Potentialunterschieden zwischen Elektroden in dem Medium umfasst, wobei jede Elektrode mit diesem Medium eine elementare Kontaktfläche aufweist, **dadurch gekennzeichnet, dass** es folgende Schritte umfasst:

   - Schätzen (510, 721, 722) einer Kontaktimpedanz mit dem Medium für jede Elektrode, wobei die Kontaktimpedanz geschätzt wird, indem die Impedanz zwischen der Elektrode und einer Gegenelektrode gemessen wird, die mit dem Medium eine Kontaktfläche aufweist, die merklich größer ist als die elementare Kontaktfläche;
   - Korrigieren (515, 731, 732) von Potentialunterschieden ausgehend von Potentialabfällen, die in den jeweiligen Kontaktimpedanzen der Elektroden auftreten;
   - Bestimmen (560, 570) von Werten der elektrischen Größe oder einer Änderung derselben an einer Mehrzahl von Punkten des betreffenden Bereichs ausgehend von den so korrigierten Potentialunterschieden.

2. Verfahren zum Bestimmen der räumlichen Verteilung einer elektrischen Größe in einem betreffenden Bereich eines Mediums, wobei das Verfahren das Durchführen von Messungen von Potentialunterschieden mittels Elektroden umfasst, die in Kontakt mit diesem angeordnet sind, wobei jede Elektrode mit diesem Medium eine elementare Kontaktfläche aufweist, **dadurch gekennzeichnet, dass** es folgende Schritte umfasst:

   - Schätzen (610, 821) einer Kontaktimpedanz mit dem Medium für jede Elektrode, wobei die Kontaktimpedanz geschätzt wird, indem die Impedanz zwischen der Elektrode und einer Gegenelektrode gemessen wird, die mit dem Medium eine Kontaktfläche aufweist, die merklich größer ist als die elementare Kontaktfläche;
   - Konstruieren eines korrigierten, direkten Modells, mit dem die Potentialunterschiede ausgehend von einer räumlichen Verteilung der elektrischen Größe in dem betreffenden Bereich oder einer Änderung derselben erhalten werden können, wobei das korrigierte, direkte Modell die Potentialabfälle in den so geschätzten Kontaktimpedanzen berücksichtigt;
   - Bestimmen (660, 860) der Werte der elektrischen Größe oder der Änderung derselben an einer Mehrzahl von Punkten des betreffenden Bereichs ausgehend von Messungen von Potentialunterschieden und des korrigierten, direkten Modells.

3. Verfahren zum Bestimmen der räumlichen Verteilung einer elektrischen Größe nach Anspruch 1 oder 2, **dadurch gekennzeichnet, dass** die Gegenelektrode aus einer Untereinheit der Mehrzahl von Elektroden gebildet ist, wobei die Untereinheit keine Elektrode umfasst, deren Kontaktimpedanz gemessen wird, wobei die Elektroden der Untereinheit mittels eines Schalters (910) mit Einfachausgang kurzgeschlossen werden.

4. Verfahren zum Bestimmen der räumlichen Verteilung einer elektrischen Größe nach Anspruch 1 oder 2, **dadurch gekennzeichnet, dass** die Gegenelektrode eine dedizierte Elektrode in einem Abstand der Elektroden ist, der merklich größer als der mittlere Abstand zwischen diesen letztgenannten ist.

5. Verfahren zum Bestimmen der räumlichen Verteilung einer elektrischen Größe nach einem der vorangehenden Ansprüche, **dadurch gekennzeichnet, dass** die Kontaktimpedanz mittels einer Impedanzspektroskopie gemessen wird.

6. Verfahren zum Bestimmen der räumlichen Verteilung einer elektrischen Größe nach Anspruch 5, **dadurch gekennzeichnet, dass** die Kontaktimpedanz ausgehend von einem Ersatzschaltungsmodell gemessen wird, das die Elektrode und das Medium des betreffenden Bereichs darstellt, wobei die Kontaktimpedanz diejenige in der Ersatzschaltung ist, mit der das Impedanzspektrum erhalten werden kann, das dem mittels der Impedanzspektroskopie gemessenen am nächsten liegt.

7. Verfahren zum Bestimmen der räumlichen Verteilung einer elektrischen Größe nach einem der vorangehenden Ansprüche, **dadurch gekennzeichnet, dass** die elektrische Größe ausgewählt ist aus der Leitfähigkeit, dem spezifischen Widerstand, der Permittivität, der Admittivität, der spezifischen Impedanz des Mediums oder einer Funktion einer dieser elektrischen Größen, einer äquivalenten Menge eines Materials, von dem eine elektrische Größe bekannt ist, oder einer Funktion dieser Menge.

8. Verfahren zum Bestimmen der räumlichen Verteilung einer elektrischen Größe nach einem der vorangehenden Ansprüche, **dadurch gekennzeichnet, dass** die Änderung der elektrischen Größe zu zwei verschiedenen Zeitpunkten aufgenommen wird.

9. Verfahren zum Bestimmen der räumlichen Verteilung einer elektrischen Größe nach einem der vorangehenden Ansprüche, **dadurch gekennzeichnet, dass** die Änderung der elektrischen Größe in zwei verschiedenen Frequenzen aufgenommen wird.

10. Verfahren zum Bestimmen der räumlichen Verteilung einer elektrischen Größe nach einem der Ansprüche 8 oder 9, **dadurch gekennzeichnet, dass** es sich um ein differenzielles TIE-Verfahren handelt.

11. Verfahren zum Bestimmen der räumlichen Verteilung einer elektrischen Größe nach einem der Ansprüche 1 bis 7, **dadurch gekennzeichnet, dass** es sich um ein TIE-Verfahren handelt.

12. Verfahren zum Bestimmen der räumlichen Verteilung einer elektrischen Größe nach einem der vorangehenden Ansprüche, **dadurch gekennzeichnet, dass** die Messungen von Potentialunterschieden zwischen Elektroden in Zweipunkt-Konfiguration erfolgen, wobei ein Strom zwischen zwei Elektroden eingeleitet wird und ein Potentialunterschied zwischen denselben Elektroden gemessen wird.

13. Verfahren zum Bestimmen der räumlichen Verteilung einer elektrischen Größe nach einem der vorangehenden Ansprüche, **dadurch gekennzeichnet, dass** eine Abbildung der elektrischen Größe oder ihrer Änderung in diesem Medium ausgehend von zuvor bestimmten Werten der elektrischen Größe konstruiert wird.


**Claims**

1. A method for determining the spatial distribution of an electrical variable in a zone of interest of a medium, said method comprising performing measurements of voltage differences between electrodes in said medium, each electrode having with this medium a unit contact area, **characterised in that**:

   - for each electrode, a contact impedance with said medium is estimated (510, 721, 722), said contact impedance being estimated by measuring the impedance between said electrode and a counter-electrode having with said medium a contact area substantially higher than said unit contact area;
   - the voltage differences are corrected (515, 731, 732) from the voltage drops occurring in the respective contact impedances of said electrodes;
   - the values of the electrical variable, or a variation in the same are determined (560, 760), at a plurality of points of said zone of interest, from the voltage differences thus corrected.

2. A method for determining the spatial distribution of an electrical variable in a zone of interest of a medium, said method comprising performing measurements of voltage differences between electrodes contacting the same, each electrode having with this medium a unit contact area, **characterised in that**:

   - for each electrode, a contact impedance with said medium is estimated (610, 821), said contact impedance being estimated by measuring the impedance between said electrode and a counter-electrode having with said medium a contact area substantially higher than said unit contact area;
   - a corrected direct model is constructed allowing said voltage differences to be obtained from a spatial distribution of the electrical variable in the zone of interest, or a variation in the same, the corrected direct model taking into account the voltage drops thus estimated in the contact impedances;
   - the values of the electrical variable, or a variation in the same are determined (660, 860), at a plurality of points of said zone of interest, from the measurements of voltage differences and the corrected direct model.

3. The method for determining the spatial distribution of an electrical variable according to claim 1 or 2, **characterised in that** the counter-electrode is formed by a subset of said plurality of electrodes, said subset not containing the electrode the contact impedance of which is measured, the electrodes of said subset being short-circuited by means of a single-output switch (910).

4. The method for determining the spatial distribution of an electrical variable according to claim 1 or 2, **characterised in that** the counter-electrode is a dedicated electrode at a distance from the electrodes which is substantially higher than the mean distance between the latter.

5. The method for determining the spatial distribution of an electrical variable according to one of the preceding claims, **characterised in that** the contact impedance is measured by means of an impedance spectroscopy.

6. The method for determining the spatial distribution of an electrical variable according to claim 5, **characterised in that** the contact impedance is measured from an equivalent circuit model representing the electrode and the medium of the zone of interest, the contact impedance being that in the equivalent circuit allowing the closest impedance spectrum to that measured by said impedance spectroscopy to be obtained.

7. The method for determining the spatial distribution of an electrical variable according to one of the preceding claims, **characterised in that** said electrical variable is chosen from conductivity, resistivity, permittivity, admittivity, impedivity of the medium, or a function of one these electrical variables, an equivalent quantity of a material an electrical variable of which is known, or a function of this quantity.

8. The method for determining the spatial distribution of an electrical variable according to one of the preceding claims, **characterised in that** the variation in the electrical variable is taken at two distinct instants.

9. The method for determining the spatial distribution of an electrical variable according to one of the preceding claims, **characterised in that** the variation in the electrical variable is taken at two distinct frequencies.

10. The method for determining the spatial distribution of an electrical variable according to one of claims 8 or 9, **characterised in that** it is a differential EIT method.

11. The method for determining the spatial distribution of an electrical variable according to one of claims 1 to 7, **characterised in that** it is an EIT method.

12. The method for determining the spatial distribution of an electrical variable according to one of the preceding claims, **characterised in that** the measurements of voltage differences between electrodes are performed in a two-point configuration, a current being injected between two electrodes and a voltage difference being measured between those same electrodes.

13. The method for determining the spatial distribution of an electrical variable according to one of the preceding claims, **characterised in that** an image of the electrical variable, or of its variation, in this medium, is constructed from the previously determined values of the electrical variable.

$110_1$
$110_{14}$
$110_2$
$100$
$110_{13}$
$110_3$
$110_{12}$
$110_4$
$130$
$110_{11}$
$110_5$
$120$
$110_{10}$
$110_6$
$110_9$
$110_7$
$140$
$110_8$

**Fig. 1**

## Fig. 2

Initialisation de la conductivité
électrique: $\sigma_0$

230

210

Distribution a priori de la
conductivité électrique
$\sigma$ dans la zone
d'intérêt

240

Injection de courant dans la
zone d'intérêt, mesure en
réponse des ddp entre
électrodes

modèle numérique

250

Vecteur de mesure
$\mathbf{U}^{meas}$
des tensions entre électrodes

Calcul des ddp entre électrodes
$\mathbf{U}^{calc}$

220

270

Calcul de la function de
coût
$\|\mathbf{U}^{meas} - \mathbf{U}^{calc}\|$

Mise à jour de
$\sigma$

260

$\widehat{\sigma}$

Temps t1, fréquence f1

Temps t2, fréquence f2

310

315

Injection de courant dans la zone d'intérêt, mesure en réponse des ddp entre électrodes

Injection de courant dans la zone d'intérêt, mesure en réponse des ddp entre électrodes

Vecteur de mesure
$$\mathbf{U}_1^{meas}$$
des tensions entre électrodes

Vecteur de mesure
$$\mathbf{U}_2^{meas}$$
des tensions entre électrodes

320

325

330

Calcul de la function de coût
$$\|\mathbf{U}_1^{meas} - \mathbf{U}_2^{meas}\|$$

350

Modèle direct

Estimation de la variation de conductivité électrique
$$\widehat{\delta\sigma}$$

340

**Fig. 3**

**Fig. 4A**

**Fig. 4B**

505 → Injection de courant et mesure des ddp entre électrodes en configuration 2 points

$I$

Initialisation de la conductivité électrique: $\sigma_0$

510 → mesure de chaque impedance de contact à l'aide d'une contre-électrode

530 → Distribution a priori de la conductivité électrique $\sigma$ dans la zone d'intérêt

515 → Correction des mesures des ddp à partir des impedances de contact

540 → modèle direct

520 → Formation d'un vecteur des ddp corrigées $\mathbf{U}^{corr}$

550 → Calcul des ddp entre électrodes $\mathbf{U}^{calc}$

560 → Calcul de la function de coût dépendant de $\|\mathbf{U}^{corr} - \mathbf{U}^{calc}\|$

570 → Mise à jour de $\sigma$

$\widehat{\sigma}$

**<u>Fig. 5</u>**

20

Initialisation de la conductivité
électrique: $\boldsymbol{\sigma_0}$

630

**Distribution a priori de la conductivité électrique $\boldsymbol{\sigma}$ dans la zone d'intérêt**

605

$I$

**Injection de courant et mesure des ddp entre électrodes en configuration 2 points**

640

**modèle direct**

610

**mesure de chaque impedance de contact à l'aide d'une contre-électrode**

645

**modèle des impedances de contact**

620

**Formation d'un vecteur des ddp mesurées**

$$\boldsymbol{U}^{meas}$$

650

**Calcul des ddp entre électrodes**

$$\boldsymbol{U}^{cc}$$

670

660

**Calcul de la function de coût dépendant de**

$$\|\boldsymbol{U}^{meas} - \boldsymbol{U}^{cc}\|$$

**Mise à jour de $\boldsymbol{\sigma}$**

$\hat{\sigma}$

**Fig. 6**

**Temps t1, fréquence f1**

**Temps t2, fréquence f2**

711 → Injection de courant et mesure des ddp entre électrodes

712 → Injection de courant et mesure des ddp entre électrodes

721 → mesure de chaque impedance de contact à l'aide d'une contre-électrode

722 → mesure de chaque impedance de contact à l'aide d'une contre-électrode

731 → Correction des mesures des ddp à partir des impedances de contact

732 → Correction des mesures des ddp à partir des impedances de contact

741 → Formation d'un vecteur des ddp corrigées $\mathbf{U}_1^{corr}$

742 → Formation d'un vecteur des ddp corrigées $\mathbf{U}_2^{corr}$

750 → Calcul de la function de coût dépendant de

$$\|\mathbf{U}_1^{corr} - \mathbf{U}_2^{corr}\|$$

770 → Modèle direct

760 → Estimation de la variation de conductivité électrique

$$\widehat{\delta\sigma}$$

**Fig. 7**

Temps t1, fréquence f1

Temps t2, fréquence f2

811 — Injection de courant et mesure des ddp entre électrodes

812 — Injection de courant et mesure des ddp entre électrodes

821 — mesure de chaque impedance de contact à l'aide d'une contre-électrode

841 — Formation d'un vecteur des ddp mesurées $\mathbf{U}_1^{meas}$

842 — Formation d'un vecteur des ddp corrigées $\mathbf{U}_2^{meas}$

850 — Calcul de la function de coût dépendant de $\|\mathbf{U}_1^{meas} - \mathbf{U}_2^{meas}\|$

870 — Modèle direct corrigé

860 — Estimation de la variation de conductivité électrique $\widehat{\delta\sigma}$

**Fig 8**

**Fig. 9**

**Fig. 10**

**Fig. 11**

**Fig. 12A**

**Fig. 12B**

**Fig. 13A**

**Fig. 13B**

## RÉFÉRENCES CITÉES DANS LA DESCRIPTION

**Littérature non-brevet citée dans la description**

- **L.M. HEIKKINEN et al.** Simultaneous reconstruction of electrode contact impedances and internai electrical properties : II. Laboratory experiments. *Meas. Sci. Techno.,* 2002, vol. 13, 1855-1861
- **J.K. SEO ; E.J. WOO.** Nonlinear inverse problems in imaging. J. Wiley & Sons
- **L.M. HEKKINEN ; T. VILHUNEN.** Détection of faults in resistive coatings with an impedance-tomography-related approach. *Meas. Sci. Technol.,* 2002, vol. 13, 865-872
- **G. BOVERMAN et al.** Methods for compensating for variable electrode contact in EIT. *IEEE Trans. on Biomedical Engineering,* Décembre 2009, vol. 56 (12), 2762-2772
- **S. HONG.** A 4.9 m$\Omega$-Sensitivity Mobile Electrical Impedance Tomography IC for Early Breast-Cancer Détection System. *IEEE Journal of Solid-State Circuits,* 2015, 245-257